(19) Europäisches Patentamt
European Patent Office
Office européen des brevets

(11) Veröffentlichungsnummer : **0 073 871**
**B1**

(12) **EUROPÄISCHE PATENTSCHRIFT**

(45) Veröffentlichungstag der Patentschrift : **26.06.85**

(51) Int. Cl.[4] : **C 07 C102/00**, C 07 C103/38, C 07 C103/48

(21) Anmeldenummer : **81810368.1**

(22) Anmeldetag : **07.09.81**

(54) **Verfahren zur Herstellung von N-substituierten N-Acetyl-2,6-dialkylanilinen.**

(43) Veröffentlichungstag der Anmeldung : **16.03.83 Patentblatt 83/11**

(45) Bekanntmachung des Hinweises auf die Patenterteilung : **26.06.85 Patentblatt 85/26**

(84) Benannte Vertragsstaaten : **AT BE CH DE FR GB IT LI LU NL SE**

(56) Entgegenhaltungen :
**EP-A- 0 017 850**
**GB-A- 2 073 173**

(73) Patentinhaber : **CIBA-GEIGY AG**
**Postfach**
**CH-4002 Basel (CH)**

(72) Erfinder : **Deglscher, Gerhard, Dr.**
**Untere Hofackerstrasse 12**
**CH-4414 Füllinsdorf (CH)**
Erfinder : **Angst, Werner, Dr.**
**Gruthweg 15**
**CH-4132 Muttenz (CH)**

**EP 0 073 871 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von N-substituierten N-Acetyl-2,6-dialkylanilinen der Formel I

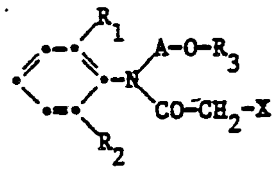

(I)

in welcher $R_1$ und $R_2$ unabhängig voneinander Methyl oder Aethyl bedeuten, A eine 1,2-Aethylengruppe, die durch eine oder zwei Methylgruppen substituiert sein kann, eine 2-Oxo-1,2-äthylengruppe oder eine 1-Methyl-2-oxo-1,2-äthylengruppe darstellt, $R_3$ für eine Alkylgruppe mit 1-3 Kohlenstoffatomen steht und X Chlor oder eine Alkoxygruppe mit 1-3 Kohlenstoffatomen bedeutet, durch Umsetzung eines N-substituierten 2,6-Dialkylanilins der Formel II

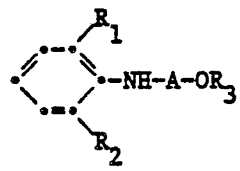

(II)

in welcher $R_1$, $R_2$, $R_3$ und A die unter Formel I angegebene Bedeutung haben, mit einem Acetylchlorid der Formel III

$$X—CH_2—CO—Cl \qquad (III),$$

in welcher X die oben angegebene Bedeutung hat.

Die N-substituierten N-Acetyl-2,6-dialkylaniline der Formel I haben eine ausgeprägte Wirkung gegen phytopathogene Mikroorganismen und werden daher zum Schutz von Kulturpflanzen gegen den Befall durch phytopathogene Mikroorganismen verwendet. N-substituierte N-Acetyl-2,6-dialkylaniline der Formel I und ihre Verwendung sind beispielsweise in den US-Patentschriften 3,952,056, 3,937,730, 4,206,228 und 4,200,451 beschrieben.

Es ist bekannt, N-substituierte N-Acetyl-2,6-dialkylaniline der Formel I durch Umsetzung der entsprechenden N-substituierten 2,6-Dialkylaniline der Formel II mit einem Acetylchlorid der Formel III herzustellen (vgl. z. B. deutsche Offenlegungsschriften 23 05 495, 23 28 340 und 25 15 091). Die Umsetzung kann sowohl in Abwesenheit als auch in Anwesenheit einer anorganischen oder organischen Base, wie Natriumhydroxid, Natriumcarbonat, Natriumbicarbonat, N,N-Dimethylanilin oder Triäthylamin durchgeführt werden. Bei der Durchführung der Acetylierung in Gegenwart von wässrigen anorganischen Basen wird jedoch ein grosser Ueberschuss an Acetylchlorid benötigt, da ein wesentlicher Teil des Acetylchlorids durch Hydrolyse verloren geht. Dieser Nachteil der Verwendung eines grossen Ueberschusses an Acetylchlorid kann bei der Verwendung von anorganischen und organischen Basen und eines inerten Lösungsmittels durch Ausschluss von Wasser vermieden werden. In diesem Fall muss jedoch das jeweils gebildete Salz durch Filtration abgetrennt und bei Verwendung eines Amins das entsprechende Amin aus dem gebildeten Amin-Hydrochlorid regeneriert werden, was einen zusätzlichen Aufwand bedeutet.

Obwohl in einzelnen Fällen Acylierungsreaktionen in Abwesenheit einer Base und in Abwesenheit eines Lösungsmittels durchgeführt werden können, ist für die Umsetzung eines N-substituierten 2,6-Dialkylanilins der Formel II mit einem Acetylchlorid der Formel III in Abwesenheit einer Base zur Erreichung einer guten Ausbeute stets die Anwesenheit eines inerten Lösungsmittels notwendig. Wird die Umsetzung in Abwesenheit eines Lösungsmittels durchgeführt, so erhält man ein dunkelgefärbtes hochviskoses Reaktionsprodukt, welches sehr schwierig zu handhaben ist und aus welchem das gewünschte Reaktionsprodukt nur in sehr mässigen Ausbeuten gewonnen werden kann.

Die Durchführung der Umsetzung eines N-substituierten 2,6-Dialkylanilins der Formel II mit einem Acetylchlorid der Formel III in einem inerten Lösungsmittel in Abwesenheit einer Base hat den Vorteil, dass der während der Umsetzung gebildete Chlorwasserstoff unmittelbar gasförmig abgetrennt werden kann. Da jedoch das Acetylchlorid aus wirtschaftlichen Gründen im wesentlichen in äquimolarer Menge verwendet wird, sind für die vollständige Umsetzung und für die vollständige Abtrennung des Chlorwasserstoffs lange Reaktionszeiten notwendig. In der Regel ist es notwendig, das Reaktionsgemisch einige Stunden auf Temperaturen von 90-110 °C zu erhitzen, wodurch Nebenreaktionen, die zur Bildung harziger Nebenprodukte führen, begüngstigt werden. Durch diese Nebenreaktionen wird die Ausbeute an Aktivsubstanz vermindert.

Die vorgenannten Verfahren sind aufgrund der ihnen anhaftenden Nachteile unwirtschaftlich. Der vorliegenden Erfindung liegt daher die Aufgabe zugrunde, ein verfahren bereitzustellen, nach dem die N-substituierten N-Acetyl-2,6-dialkylaniline der Formel I auf einfache und wirtschaftliche Weise in guten Ausbeuten in technischem Masstab hergestellt werden können.

Es wurde nun gefunden, dass man N-substituierte N-Acetyl-2,6-dialkylaniline der Formel I in ausgezeichneter Ausbeute und ausgezeichneter Reinheit herstellen kann, wenn man die Umsetzung des N-substituierten 2,6-Dialkylanilins der Formel II mit dem Acetylchlorid der Formel III in überschüssigem Acetylchorid der Formel III asl Lösungsmittel durchführt. Das erfindungsgemässe Verfahren zur Herstellung von N-substituierten N-Acetyl-2,6-dialkylanilinen der Formel I ist daher dadurch gekennzeichnet, dass man das N-substituierte 2,6-Dialkylanilin der Formel II mit dem Acetylchlorid der Formel III in Gegenwart von überschüssigem Acetylchlorid der Formel III als Lösungsmittel durchführt, das überschüssige Acetylchlorid der Formel III durch Destillation abtrennt, das gebildete N-substituierte N-Acetyl-2,6-dialkylanilin der Formel I mit Wasser neutral wäscht und anschliessend trocknet.

Das Acetylchlorid der Formel III wird in der Regel in einer Menge von 2-20 Mol pro Mol N-substituiertes 2,6-Dialkylanilin der Formel II verwendet. Vorzugsweise verwendet man das Acetylchlorid der Formel III in einer Menge von 4-8 Mol pro Mol N-substituiertes 2,6-Dialkylanilin der Formel II. Die Umsetzung wird vorteilhaft im Temperaturbereich von 70 °C bis Rückflusstemperatur des Reaktionsgemisches durchgeführt. Vorzugsweise führt man die Umsetzung bei Rückflusstemperatur des Reaktionsgemisches durch.

Das Waschen des nach der Abtrennung des überschüssigen Acetylchlorids der Formel III mit Wasser kann bei 50-110 °C durchgeführt werden. Es ist vorteilhaft, den Waschvorgang einige Male zu wiederholen. Weiterhin ist es vorteilhaft, dem Wachwasser eine Menge Alkali zuzusetzen, die ausreicht, um den pH-Wert des Wassers auf 4-10 zu stellen. Als Alkali, das dem Waschwaser zugesetzt werden kann, ist insbesondere Natriumhydroxid oder Kaliumhydroxid geeignet. Anschliessend wird das Produkt gegebenenfalls nach einer weiteren Wäsche mit reinem Wasser durch Erhitzen auf 100-120 °C im Vakuum getrocknet.

Das erfindungsgemässe Verfahren kann diskontinuierlich oder kontinuierlich durchgeführt werden.

Bei der diskontinuierlichen Durchführung des Verfahrens geht man vorteilhaft so vor, dass man das N-substituierte 2,6-Dialkylanilin der Formel II in siedendes Acetylchlorid der Formel III einträgt und das überschüssige Acetylchlorid nach beendigter Umsetzung im Vakuum abdestilliert.

Bei der kontinuierlichen Durchführung des Verfahrens ist es zweckmässig, das Acetylchlorid der Formel III und das N-substituierte 2,6-Dialkylanilin der Formel II bei Reaktionstemperatur gleichzeitig in den Reaktor einzuführen und das Gemisch unmittelbar anschliessend im Vakuum einzudampfen. Die kontinuierliche Durchführung des Verfahrens kann vorteilhaft in einem Kaskadenreaktor von 2 bis 4 Stufen oder in einem Fallfilmreaktor vorgenommen werden.

Der bei der Reaktion gebildete Chlorwasserstoff wird gasförmig abgetrennt und kann entweder komprimiert und in Stahlflaschen abgefüllt oder in Wasser zu konzentrierter Salzsäure gelöst oder durch Oxydation zu Chlor verarbeitet werden.

Unter den als Ausgangsmaterialien verwendeten N-substituierten 2,6-Dialkylanilinen der Formel II sind diejenigen bevorzugt, in welchen A eine 1,2-Aethylengruppe, eine 1-Methyl-1,2-Aethylengruppe oder eine 1-Methoxy-2-oxo-1,2-äthylengruppe und $R_3$ Methyl bedeutet. Besonders bevorzugte Ausgangsmaterialien der Formel III sind N-(1'-Methyl-2'-methoxyäthyl)-2-äthyl-6-methylanilin und N-(1'-Methoxycarbonyläthyl)-2,6-dimethylanilin. Von den Acetylchloriden der Formel III sind Chloracetylchlorid und Methoxyacetylchlorid bevorzugt.

Es ist ein besonderer Vorteil des erfindungsgemässen Verfahrens, dass die Belastung der Umwelt mit Schadstoffen auf ein Minimum reduziert ist. Die bei Durchführung des erfindungsgemässen Verfahrens erhaltenen Produkte sind sehr rein und die Ausbeute ist hervorragend. Da die Umsetzung der N-substituierten 2,6-Dialkylaniline der Formel II mit den Acetylchloriden der Formel III sehr rasch vonstatten geht, können die Verweilzeiten von Ausgangs- und Endprodukten unter Reaktionsbedingungen sehr gehalten werden. Als Folge davon kann die Grösse des Reaktors für eine vorgegebene Menge an Produkt relativ klein gehalten werden. Da das überschüssige Acetylchlorid der Formel III nach seiner Abtrennung erneut in die Reaktion eingesetzt werden kann, werden Substanzverluste weitgehend vermieden. Ein weiterer Vorteil des erfindungsgemässen Verfahrens besteht schliesslich darin, dass das Mengenverhältnis der Reaktanden in sehr weiten Grenzen variiert werden kann, ohne dass die Qualität des Endproduktes nachteilig beeinflusst wird.

Mit dem erfindungsgemässen Verfahren wird es daher möglich, die N-substituierten N-Acetyl-2,6-dialkylaniline der Formel I auf einfache Weise in ausgezeichnet Ausbeute und Reinheit herzustellen.

Das erfindungsgemässe Verfahren wird durch die nachfolgenden Beispiele näher erläutert.

## Beispiel 1

Herstellung von N-(2'-Methoxy-1'-methyläthyl)-N-Chloracetyl-2-Aethyl-6-methylanilin

In einem mit Rührer, Tropftrichter und Kühler versehenen Dreiliterdreihalskolben werden 1 356 g (12

Mol) Chloracetylchlorid vorgelegt und zum Sieden erhitzt. Dann werden 207 g (1 Mol) N-(2'-Methoxy-1'-methyläthyl)-2-äthyl-6-methylanilin mit einer solchen Geschwindigkeit zugetropft, dass die Lösung immer gleichmässig am Sieden bleibt und Gas entwickelt. Nach Zugabe des N-(2'-Methoxy-1'-methyläthyl)-2-äthyl-6-methylanilins wird der Ueberschuss an Chloracetylchlorid in einem Rotationsverdampfer bei einem Druck von 2 kPa (15 Torr) abdestilliert. Das so erhaltene Rohprodukt wird in einem Scheidetrichter mit 200 ml Wasser gewaschen, wobei der pH-Wert des Waschwassers durch Zugabe von 30 %iger Natronlauge auf 8 eingestellt wird. Nach Abtrennung der wässrigen Phase wird das Produkt zunächst noch zweimal mit reinem Wasser gewaschen und dann 30 min bei 110 °C und 2 kPa (15 Torr) getrocknet. Es werden so 273,8 g (96,6 % d. Th.) N-(2'-Methoxy-1'-methyläthyl)-N-chloracetyl-2-äthyl-6-methylanilin erhalten.

Beispiel 2

Herstellung von N-(1'-Methoxycarbonyläthyl)-N-methoxyacetyl-2,6-dimethylanilin

In einem mit Rührer, Abtropftrichter und Kühler versehenen Zweiliterdreihalskolben werden 868 g (8 Mol) Methoxyacetylchlorid vorgelegt und zum Sieden erhitzt. Dann werden 165,6 g (0,8 Mol) N-(1'-Methoxycarbonyläthyl)-2,6-dimethylanilin mit solcher Geschwindigkeit zugetropft, dass die Lösung immer gleichmässig am Sieden bleibt und Gas entwickelt. Nach Zugabe des N-(1'-Methoxycarbony-läthyl)-2,6-Dimethylanilins wird das überschüssige Methoxyacetylchlorid in einem Rotationsverdampfer bei einem Druck von 2 kPa (15 Torr) abdestilliert. Da so erhaltene Rohprodukt wird bei 80 °C mit 100 ml Wasser gewaschen, wobei der pH-Wert des Waschwassers durch Zugabe von 1N-Natronlauge auf 4 eingestellt wird. Nach Abtrennung der wässrigen Phase wird das Produkt noch zweimal mit warmem Wasser gewaschen und dann 1 Stunde bei 110 °C und 2 kPa (15 Torr) getrocknet. Es werden so 217,3 g (97,2 % d. Th.) N-(1'-Methoxycarbonyläthyl)-N-methoxyacetyl-2,6-dimethylanilin erhalten.

**Patentansprüche**

1. Verfahren zur Herstellung von N-substituierten N-Acetyl-2,6-dialkylanilinen der Formel I

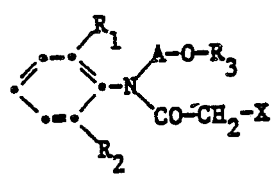

(I)

in welcher $R_1$ und $R_2$ unabhängig voneinander Methyl oder Aethyl bedeuten, A eine 1,2-Aethylengruppe, die durch eine oder zwei Methylgruppen substituiert sein kann, eine 2-oxo-1,2-Aethylengruppe oder eine 1-Methyl-2-oxo-1,2-äthylengruppe darstellt, $R_3$ für eine Alkylgruppe mit 1-3 Kohlenstoffatomen steht und X Chlor oder eine Alkoxygruppe mit 1-3 Kohlenstoffatomen bedeutet, durch Umsetzung eines N-substituierten-2,6-Dialkylanilins der Formel II

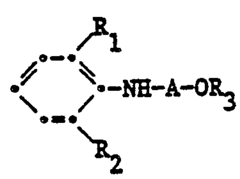

(II)

in welcher $R_1$, $R_2$, $R_3$ und A die unter Formel I angegebene Bedeutung haben, mit einem Acetylchlorid der Formel III

$$X—CH_2—CO—Cl \quad (III)$$

in welcher X die unter Formel I angegebene Bedeutung hat, dadurch gekennzeichnet, dass man die Umsetzung des N-substituierten 2,6-Dialkylanilins der Formel II mit dem Acetylchlorid der Formel III in Gegenwart von überschüssigem Acetylchlorid der Formel III als Lösungsmittel durchführt, das überschüssige Acetylchlorid der Formel III anschliessend durch Destillation abtrennt, das erhaltene N-substituierte N-Acetyl-2,6-dialkylanilin der Formel I mit Wasser neutral wäscht und anschliessend trocknet.

2. Verfahren nach Anspruch 1, dadurch gekennzeichnet, dass man pro Mol N-substituiertes 2,6-Dialkylanilin der Formel II 2-20 Mol Acetylchlorid der Formel III verwendet.

3. Verfahren nach Anspruch 1, dadurch gekennzeichnet, dass man pro Mol N-substituiertes 2,6-Dialkylanilin der Formel II 4-8 Mol Acetylchlorid der Formel III verwendet.

4. Verfahren nach Anspruch 1, dadurch gekennzeichnet, dass man die Umsetzung des N-substituierten 2,6-Dialkylanilins der Formel II mit dem Acetylchlorid der Formel III im Temperaturbereich von 70 °C bis Rückflusstemperatur des Reaktionsgemisches durchführt.

5. Verfahren nach Anspruch 1, dadurch gekennzeichnet, dass man die Umsetzung des N-substituierten 2,6-Dialkylanilins der Formel II mit dem Acetylchlorid der Formel III bei Rückflusstemperatur des Reaktionsgemisches durchführt.

6. Verfahren nach Anspruch 1, dadurch gekennzeichnet, dass man das nach Abtrennung des überschüssigen Acetylchlorids der Formel III erhaltene N-substituierte N-Acetyl-2,6-dialkylanilin in der Formel I bei 50 bis 110 °C mit Wasser wäscht.

7. Verfahren nach Ansprüchen 1 und 6, dadurch gekennzeichnet, dass man den pH-Wert des Waschwassers durch Zugabe von Alkali auf 4-10 einstellt.

**Claims**

1. Process for the preparation of an N-substituted N-acetyl-2,6-dialkylaniline of the formula I

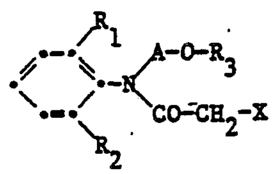

(I)

in which each of $R_1$ and $R_2$ independently of the other is methyl or ethyl, A is a 1,2-ethylene group which can be substituted by 1 or 2 methyl groups, or is a 2-oxo-1,2-ethylene group or a 1-methyl-2-oxo-1,2-ethylene group, $R_3$ is an alkyl group having 1 to 3 carbon atoms, and X is chlorine or an alkoxy group having 1 to 3 carbon atoms, by reacting an N-substituted 2,6-dialkylaniline of the formula II

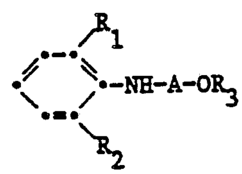

(II)

in which $R_1$, $R_2$, $R_3$ and A are as defined for formula I, with an acetyl chloride of the formula III

$$X—CH_2—CO—Cl \quad (III)$$

in which X is as defined for formula I, which process comprises carrying out the reaction of N-substituted 2,6-dialkylaniline of the formula II with the acetyl chloride of the formula III in the presence of excess acetyl chloride of the formula III as solvent, subsequently distilling off the excess acetyl chloride of the formula III, washing the resultant N-substituted N-acetyl-2,6-dialkylaniline of the formula I with water until neutral, and subsequently drying the product.

2. A process according to claim 1, wherein 2 to 20 mol of acetyl chloride of the formula III are used per mol of N-substituted 2,6-dialkylaniline of the formula II.

3. A process according to claim 1, wherein 4 to 8 mol of acetyl chloride of the formula III are used per mol of N-substituted 2,6-dialkylaniline of the formula II.

4. A process according to claim 1, wherein the reaction of N-substituted 2,6-dialkylaniline of the formula II with the acetyl chloride of the formula III is carried out in the temperature range from 70 °C to the reflux temperature of the reaction mixture.

5. A process according to claim 1, wherein the reaction of N-substituted 2,6-dialkylaniline of the formula II with the acetyl chloride of the formula III is carried out at the reflux temperature of the reaction mixture.

6. A process according to claim 1, wherein the N-substituted N-acetyl-2,6-dialkylamine of the formula I, obtained after removal by distillation of the excess acetyl chloride of the formula III, is washed with water at 50° to 110 °C.

7. A process according to either of claims 1 or 6, wherein the pH value of the wash water is adjusted to 4-10 by adding alkali.

**Revendications**

1. Procédé de préparation de N-acétyl-2,6-dialkylanilines substituées à l'azote et répondant à la formule

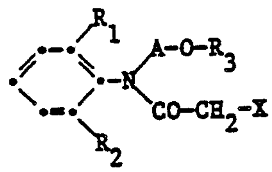 (I)

dans laquelle $R_1$ et $R_2$ représentent indépendamment l'un de l'autre un groupe méthyle ou éthyle, A représente un groupe 1,2-éthylène qui peut être substitué par un ou deux groupes méthyle, un groupe 2-oxo-1,2-éthylène ou un groupe 1-méthyl-2-oxo-1,2-éthylène, $R_3$ représente un groupe alkyle en C 1-C 3 et X représente le chlore ou un groupe alcoxy en C 1-C 3, par réaction d'une 2,6-dialkylaniline substituée à l'azote et répondant à la formule II

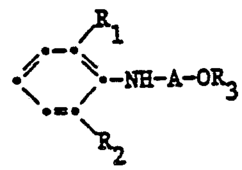 (II)

dans laquelle $R_1$, $R_2$, $R_3$ et A ont les significations indiquées en référence à la formule I, avec un chlorure d'acétyle de formule III

$$X—CH_2—CO—Cl \quad (III)$$

dans laquelle X a la signification indiquée en référence à la formule I, caractérisé en ce que l'on fait réagir la 2,6-dialkylaniline substituée à l'azote et répondant à la formule II avec le chlorure d'acétyle de formule III en présence d'un excès du chlorure d'acétyle de formule III qui sert de solvant, on sépare ensuite l'excès du chlorure d'acétyle de formule III par distillation, on lave la N-acétyl-2,6-dialkylaniline substituée à l'azote et répondant à la formule I ainsi obtenue par l'eau jusqu'à neutralité puis on la sèche.

2. Procédé selon la revendication 1, caractérisé en ce que l'on utilise de 2 à 20 moles du chlorure d'acétyle de formule III par mole de la 2,6-dialkylaniline substituée à l'azote et répondant à la formule II.

3. Procédé selon la revendication 1, caractérisé en ce que l'on utilise de 4 à 8 moles du chlorure d'acétyle de formule III par mole de la 2,6-dialkylaniline substituée à l'azote et répondant à la formule II.

4. Procédé selon la revendication 1, caractérisé en ce que l'on fait réagir la 2,6-dialkylaniline substituée à l'azote et répondant à la formule II avec le chlorure d'acétyle de formule III dans un intervalle de température allant de 70 °C jusqu'à la température de reflux du mélange de réaction.

5. Procédé selon la revendication 1, caractérisé en ce que l'on fait réagir la 2,6-dialkylaniline substituée à l'azote et répondant à la formule II avec le chlorure d'acétyle de formule III à la température de reflux du mélange de réaction.

6. Procédé selon la revendication 1, caractérisé en ce que l'on lave la N-acétyl-2,6-dialkylaniline substituée à l'azote et répondant à la formule I obtenue après séparation de l'excès du chlorure d'acétyle de formule III, par l'eau à une température de 50 à 110 °C.

7. Procédé selon les revendications 1 et 6, caractérisé en ce que l'on règle le pH de l'eau de lavage à une valeur de 4 à 10 par addition d'un alcali.